# EUROPEAN PATENT APPLICATION

(11) **EP 3 698 729 A1**
(43) Date of publication of application: **26.08.2020**
(21) Application number: 17928917.8
(22) Date of filing: 17.10.2017
(51) Int. Cl.: A61B 17/12

(54) **LEFT ATRIAL APPENDAGE OCCLUSION DEVICE AND LEFT ATRIAL APPENDAGE OCCLUSION SYSTEM**

(71) Applicant: Shanghai MicroPort Medical (Group) Co., Ltd., Shanghai 201203 (CN)
(72) Inventor: WANG, Zhen, Shanghai 201203 (CN); YAO, Yao, Shanghai 201203 (CN); ZHOU, Yi, Shanghai 201203 (CN); LI, Junfei, Shanghai 201203 (CN)
(74) Representative: Patentship Patentanwaltsgesellschaft mbH
(86) International application number: PCT/CN2017/106563
(87) International publication number: WO 2019/075640

(57) **Abstract**

Disclosed are a left atrial appendage occlusion device (100, 300) and a left atrial appendage occlusion system. A connecting member (140, 240, 340, 440) comprises a first unit (140a, 240a, 440a) and a second unit (140b, 240b, 440b), wherein the first unit (140a, 240a, 440a) is connected to a first frame (110, 310, 410), the second unit (140b, 240b, 440b) is connected to a second frame (120, 320, 420), and the first unit (140a, 240a, 440a) is rotatably connected to the second unit (140b, 240b, 440b). Alternatively, the connecting member (140, 240, 340, 440) comprises the first unit (140a, 240a, 440a), the second unit (140b, 240b, 440b) and a third unit (140c, 240c), wherein the first unit (140a, 240a, 440a) is connected to the first frame (110, 310, 410), the second unit (140b, 240b, 440b) is connected to the second frame (120, 320, 420), the first unit (140a, 240a, 440a) is rotatably connected to the third unit (140c, 240c), and the second unit (140b, 240b, 440b) is rotatably connected to the third unit (140c, 240c). Thereby, with regard to left atrial appendages having lumens of different shapes, the relative positions of the first frame (110, 310, 410) and the second frame (120, 320, 420) can be changed by means of the connecting member (140, 240, 340, 440), i.e., the shape of the left atrial appendage occlusion device (100, 300) is adjusted so as to be capable of being adapted to left atrial appendages having lumens of different shapes.

## Description

### TECHNICAL FIELD

The present application relates to the field of medical instruments and, particularly, to a left atrial appendage occluder and a left atrial appendage occlusion system.

### BACKGROUND

Atrial Fibrillation (AF) is the common continuous arrhythmia in clinic. The incidence of AF in the general population is 0.5-1.3%. The main hazard of AF is to promote thrombosis. Complications such as stroke and peripheral vascular embolization caused by thrombus detachment significantly increase the disability and fatality rate. Stroke is the most common and devastating complication of AF. About 15 million people worldwide suffer from stroke each year, 20-25% of which are attributed to AF. The study suggests that 60% of patients with rheumatic heart disease have a cardiogenic thrombus from the left atrial appendage, and more than 90% of thrombi in the patients with non-valvular AF are formed in the left atrial appendage. Therefore, the intervention of the left atrial appendage to prevent thromboembolism in patients with AF, especially stroke, has important theoretical and clinical significance.

Anticoagulant therapy is a standard method for preventing complications of stroke and AF at present, but has certain limitations. Therefore, the use of safer and more effective measures to prevent AF and stroke is of great significance. At present, medical interventions are mostly used to occlude the left atrial appendage. The commonly used left atrial appendage occluders comprise a single plug type occluder represented by WATCHMAN and a plug-and-disc type left atrial appendage occluder represented by AMPLATZER Cardiac Plug (ACP).

### 1. Deficiencies and disadvantages of plug type left atrial appendage occluder

The plug type left atrial appendage occluder has a nickel-titanium alloy frame with a self-expanding structure and barbs fixed around. The surface of the occluder towards atrial is covered with a porous polytetrafluoroethylene membrane for blood inflow outflow in the left atrial appendage.

When the plug type left atrial appendage occluder has been plugged into the left atrial appendage, the entrance of left atrial appendage cannot be completely occluded since the shape of the entrance of the left atrial appendage is irregular, and the occluder has a limited deformability. Therefore, it is difficult to eliminate the thrombus caused by leakage of the left atrial appendage due to AF. The left atrial appendage has different structures and depths, and in addition, a multi-cavity structure. The plug type occluder is unable to fully adapt to the anatomical structures of all left atrial appendages as well as to achieve a stable fixation.

### 2. Deficiencies and disadvantages of the plug-and-disc type left atrial appendage occluder

The plug-and-disc type left atrial appendage occluder is a two-disc type device consisting of a butterfly blade which would be placed in the left atrial appendage and a butterfly cap. The butterfly blade and the butterfly cap are connected by a concave waist. The butterfly blade is placed in the left atrial appendage to prevent the occluder from shifting. The butterfly cap seals the entrance of the left atrial appendage.

The plug-and-disc type left atrial appendage occluder has a structure in which the sealing disc and the fixed disc are integrated, and cannot be completely deformed independently. After the plug is plugged into the left atrial appendage, the disc portion adhered to the entrance of the left atrial appendage is subjected to the pull of the plug portion, so that the disc portion cannot fully fit to the entrance of the left atrial appendage, and thus it is difficult to achieve an optimal occluding effect. Moreover, since the length adjustments of the plug portion and the disc portion are limited, it is difficult to achieve the optimal fixation and blood flow blockage, and the disc structure does not adapt to different lumen shapes of the left atrial appendage.

In short, the plug type left atrial appendage occluder or plug-and-disc type left atrial appendage occluder is unable to adapt various shapes of the left atrial appendage and difficult to attain the optimal fixation due to a limited adjustability.

### SUMMARY

It is an object of the present application to provide a left atrial appendage occluder and a left atrial appendage occlusion system for solving the problem that the existed left atrial appendage occluders are unable to adapt various shapes of the left atrial appendage.

To solve the above technical problem, present disclosure provides a left atrial appendage occluder, including:
a first frame configured to delimit a substantially closed space with the left atrial appendage;
a second frame comprising an anchoring structure; and
a connecting member comprising a first element and a second element, the first element connected to the first frame, the second element connected to the second frame, the first element is rotatably connected to the second element.

Optionally, in the left atrial appendage occluder, the first element forms a receiving volume, in which the second element is partially received to be rotatable relative to the first element; or, the second element forms a receiving volume, in which the first element is partially received to be rotatable relative to the second element.

Optionally, in the left atrial appendage occluder, each or either of the first element and the second element is made of a biocompatible polymer material.

Optionally, in the left atrial appendage occluder, the biocompatible polymer material is at least one selected from the group consisting of polysulfone, polyphenylene sulfone, polyetheretherketone, elastic polyurethane, silicone rubber and polyethylene glycol.

Optionally, in the left atrial appendage occluder, each or either of the first element and the second element has at least an arc section, a slot or a hollow structure to allow an axial bending.

The present disclose still provides a left atrial appendage occluder, including:
a first frame configured to delimit a substantially closed space in the left atrial appendage;
a second frame having an anchoring structure fixed on an exterior surface thereof; and
a connecting member comprising a first element, a second element and a third element, the first element connected to the first frame, the second element connected to the second frame, a connection between the first element and the third element being rotatable, the second element is rotatably connected to the third element.

Optionally, in the left atrial appendage occluder, the third element forms a first receiving volume and a second receiving volume, the first element partially received in the first receiving volume, the second element partially received in the second receiving volume, each of the first element and the second element being rotatable with respect to the third element; or, the first element forms a first receiving volume and the second element forms a second receiving volume, opposing ends of the third element received in the first receiving volume and the second receiving volume, respectively.

Optionally, each of the first receiving volume and the second receiving volume is a ball cup, in which a corresponding ball pin mating with the ball cup is received.

Optionally, in the left atrial appendage occluder, one or more of the first element, the second element and the third element is made of a biocompatible polymer material.

Optionally, in the left atrial appendage occluder, the biocompatible polymer material is at least one selected from the group consisting of polysulfone, polyphenylene sulfone, polyetheretherketone, elastic polyurethane, silicone rubber and polyethylene glycol.

Optionally, in the left atrial appendage occluder, one or more of the first element, the second element and the third element has at least a section comprising an arc section, a slot or a hollow structure to allow an axial bending.

Optionally, in the left atrial appendage occluder, each of a connecting section between the third element and the first element and a connecting section between the third element and the second element has a greater radial dimension than a non-connecting section of the third element.

Optionally, in the left atrial appendage occluder, a surface of the first frame is covered with a polymer membrane and/or a surface of the second frame is covered with a polymer membrane.

The present disclosure also provides a left atrial appendage occlusion system, including: a delivery catheter, a pusher, and a left atrial appendage occluder as defined above, the delivery catheter configured to establish a delivery channel, the pusher configured to push the left atrial appendage occluder along the delivery channel.

In the left atrial appendage occluders and left atrial appendage occlusion system provided in present disclosure, the connecting member includes the first element and the second element, the first element connected to the first frame, the second element connected to the second frame, the first element is rotatably connected to the second element; or, the connecting member includes the first element, the second element and the third element, the first element connected to the first frame, the second element connected to the second frame, the first element is rotatably connected to the third element, the second element is rotatably connected to the third element. In this way, when facing left atrial appendages with various shapes, relative position between the first frame and the second frame is able to be changed by the connecting member. That is, the configuration of the left atrial appendage occluder is able to be adjusted, so as to adapt various lumen shapes of the left atrial appendages.

### BRIEF DESCRIPTION OF THE DRAWINGS

Fig. 1 is a structural schematic diagram of a left atrial appendage occluder according to embodiment 1 of the present disclosure.
Fig. 2 is a structural schematic diagram of a third element according to embodiment 1 of the present disclosure.
Fig. 3 is a structural schematic diagram of a connecting member according to embodiment 1 of the present disclosure.
Fig. 4 schematically shows a state of the left atrial appendage occluder according to embodiment 1 of the present disclosure covering a normal left atrial appendage.
Fig. 5 schematically shows a state of the left atrial appendage occluder according to embodiment 1 of the present disclosure covering a short chicken wing left atrial appendage.
Fig. 6 schematically shows a state of the left atrial appendage occluder according to embodiment 1 of the present disclosure covering a multi-cavity left atrial appendage.
Fig. 7 is a structural schematic diagram of a third element according to embodiment 2 of the present disclosure.
Fig. 8 is a structural schematic diagram of a connecting member according to embodiment 2 of the present disclosure.
Fig. 9 is a structural schematic diagram of a left atrial appendage occluder according to embodiment 3 of the present disclosure.

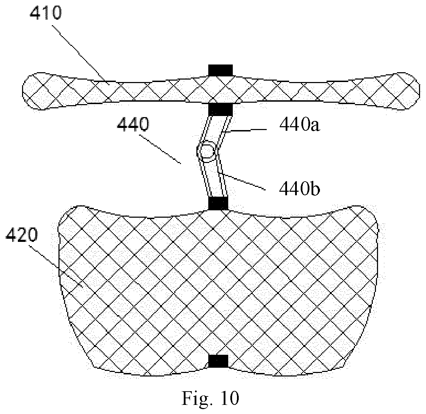

Fig. 10 is a structural schematic diagram of a left atrial appendage occluder according to embodiment 4 of the present disclosure.

In the figures,
- 10;: left atrial appendage;
- 100;: left atrial appendage occluder;
- 110;: first frame;
- 111;: first fixation structure;
- 112;: first frame strip;
- 113;: third fixation structure;
- 120;: second frame;
- 121;: second fixation structure;
- 122;: second frame strip;
- 123;: fourth fixation structure;
- 130;: anchoring structure;
- 140;: connecting member;
- 140a;: first element;
- 140b;: second element;
- 140c;: third element;
- 141;: first portion;
- 142;: second portion;
- 143;: third portion;
- 1410;: first receiving volume;
- 1420;: second receiving volume;
- 1430;: the other end of a neck portion of the first element;
- 1431;: the other end of a neck portion of the second element;
- 240;: connecting member;
- 240a;: first element;
- 240b;: second element;
- 240c;: third element;
- 241;: first portion;
- 242;: second portion;
- 243;: third portion;
- 2410;: first receiving volume;
- 2420;: second receiving volume;
- 300;: left atrial appendage occluder;
- 310;: first frame;
- 320;: second frame;
- 321;: second fixation structure;
- 322;: second frame strip;
- 330;: anchoring structure;
- 340;: connecting member;
- 410;: first frame;
- 420;: second frame;
- 440;: connecting member;
- 440a;: first element; and
- 440b;: second element.

### DETAILED DESCRIPTION

Specific embodiments of the left atrial appendage occluders and left atrial appendage occlusion system provided in present disclosure will be described below in further detail with reference to the accompanying drawings. Features and advantages of the disclosure will be more apparent from the following description and appended claims. It is noted that figures are provided in a very simplified form not necessarily presented to scale, with their only intention to facilitate convenience and clarity in explaining the disclosed embodiments. Moreover, the structures shown in figures are often parts of actual structures. In particular, figures are sometimes drawn to different scales in order to give emphasis on different details.

All numerical values that appear in this disclosure are presumed to be modified by the term "about", whether or not explicitly indicated. The term "about", in the context of a numerical value, generally refers to that one of skill in the art would consider the numerical value is equivalent to the recited numerical value (i.e., having the same function or result) and a neighboring range thereof. In many instances, the term "about" may include numbers that are rounded to the nearest significant figures. Other uses of the term "about" (i.e., in a context other than numeric values) may be assumed to have their ordinary and customary definitions, as understood from and consistent with the context of the specification, unless otherwise specified.

As used herein, "proximal end" or "distal end" refers to the relative orientation, relative position, or direction of elements or actions that are relative to each other from the perspective of an operator operating the device. Yet without wishing to be limiting in any sense, the "proximal end" generally refers to the end of the medical device close to the operator during its normal operation, while the "distal end" generally refers to the end that enters into the body of the patient first.

As used herein, the term "axial" refers to the direction of a central axis of the left atrial appendage occluder or the direction of a central axis of a constituent component of the left atrial appendage occluder, while the term "radial" refers to the direction perpendicular to the central axis of the left atrial appendage occluder or the direction perpendicular to a central axis of a constituent component of the left atrial appendage occluder.

In order to overcome the problem in the prior art that it is difficult to achieve a good fixation due to significant variations in the lumen shapes of left atrial appendages, the present disclosure provides a left atrial appendage occluder, the connecting member of which allows rotation in three degrees of freedom between a first frame (herein, the first frame is configured essentially for occlusion function and can also be referred to as an "occluding disc") and a second frame (herein, the second frame is configured essentially for anchoring function and can also be referred to as an "anchoring disc"), enabling greater angular adjustments as compared to the springs used in prior art.

The left atrial appendage occluder provided in present disclosure includes: the first frame that delimits a substantially closed space with the left atrial appendage to restrict the flow of blood into and out of the left atrial appendage; a second frame that has an anchoring structure fixed on an exterior surface thereof; and a connecting member including a first element and a second element, the first element connected to the first frame, the second element connected to the second frame, the first element is rotatably connected to the second element. Herein, the connection includes both direct connection and indirect connection. That is, other components may be provided between the first element and first frame. In this way, the connecting member allows relative positional changes between the first frame and the second frame, i.e. allowing shape adjustments of the left atrial appendage to adapt various lumen shapes of left atrial appendages.

### Embodiment 1

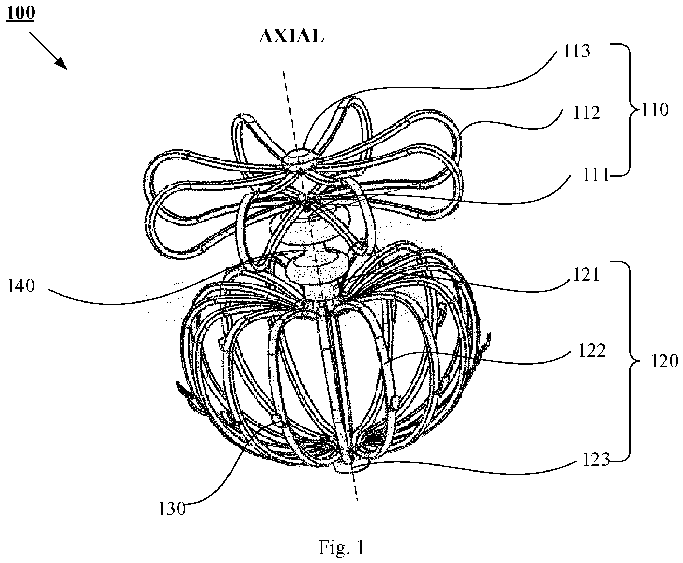

Reference is now made to FIG. 1, a structural schematic diagram of a left atrial appendage occluder according to embodiment 1 of the present disclosure. As shown in FIG. 1, the left atrial appendage occluder 100 includes: a first frame 110, the surface of which is covered with a polymer membrane (not shown in FIG. 1); a second frame 120, the exterior surface of which is attached with an anchoring structure 130; and a connecting member 140 that connects the first frame 110 and the second frame 120 in such a manner that allows relative positional changes between the first frame 110 and the second frame 120.

In this embodiment, the connecting member 140 is bendable axially. When the connecting member 140 is bended axially, relative positional changes between the first frame 110 and the second frame 120 includes: mutual approaching of the first frame 110 and the second frame 120 at one side of the left atrial appendage occluder 100 and mutual moving away of the first frame 110 and the second frame 120 at the other side of the left atrial appendage occluder 100. As a result, the overall shape of the left atrial appendage occluder 100 is able to be adjusted to adapt various lumen shapes of left atrial appendages through axial bending of the connecting member 140.

In this embodiment, the first frame 110 and the covering polymer membrane may together serve as an occlusion structure to occlude the left atrial appendage. The polymer membrane may cover either an exterior or interior surface of the first frame 110. The first frame 110 may be fabricated by weaving or cutting. In addition, the material of the first frame 110 may be metallic material and/or degradable polymer material.

The second frame 120 and the anchoring structure 130 attached to the exterior surface thereof can together serve as a fixation structure to secure the left atrial appendage occluder 100 to the left atrial appendage. Likewise, the second frame 120 may be fabricated by weaving or cutting. Further, the material of the second frame 120 may be metallic material and/or degradable polymer material. Preferably, an interior or exterior surface of the second frame 120 is covered with a polymer membrane. In this case, the second frame 120, together with the covering polymer membrane, can additionally play a role in occluding the left atrial appendage to achieve a better occlusion performance of the left atrial appendage occluder 100.

The anchoring structure 130 may be either integratedly formed with the second frame 120 or fixed onto the second frame 120 by welding. Preferably, the anchoring structure 130 includes a plurality of barbs distributed uniformly across the exterior surface of the second frame 120. Further, the plurality of barbs are arranged on a single circumference line of the exterior surface of the second frame 120.

Further, the first frame 110 includes a first fixation structure 111 and a plurality of first frame strips 112 fixedly attached to the first fixation structure 111. Connection between the first fixation structure 111 and the connecting member 140 is either fixable or movable. For example, the first fixation structure 111 is rotatable axially and/or radially relative to the connecting member 140. With continued reference to FIG. 1, in this embodiment, the first frame 110 further includes a third fixation structure 113 located at a proximal end of the first frame 110. In this case, the first fixation structure 111 is located at a distal end of the first frame 110, and the plurality of first frame strips 112 are also fixedly attached to the third fixation structure 113. The plurality of first frame strips 112 diverge from the first fixation structure 111 and then converge to the third fixation structure 113. Here, the first frame 110 is disc-shaped.

The second frame 120 includes a second fixation structure 121 and a plurality of second frame strip 122 fixedly attached to the second fixation structure 121. Connection between the second fixation structure 121 and the connecting member 140 is either fixable or movable. For example, the second fixation structure 121 is rotatable axially and/or radially relative to the connecting member 140. With continued reference to FIG. 1, in this embodiment, the second frame 120 further include a fourth fixation structure 123 located at a distal end of the second frame 120. In this case, the second fixation structure 121 is located at a proximal end of the second frame 120, and the plurality of second frame strip 122 may be also fixedly attached to the fourth fixation structure 123. The plurality of second frame strip 122 diverge from the second fixation structure 121 and then converge to the fourth fixation structure 123. Here, the second frame 120 is cage-shaped. Further, the second frame 120 has a radial dimension less than the first frame 110. In other words, the second frame 120 may be relatively thin and long, while the first frame 110 may be relatively thick and short. In such a way, the left atrial appendage occluder 100 is able to better adapt various lumen shapes of left atrial appendages, i.e., being able to adapt both a left atrial appendage with a large lumen opening and a left atrial appendage with a small lumen opening.

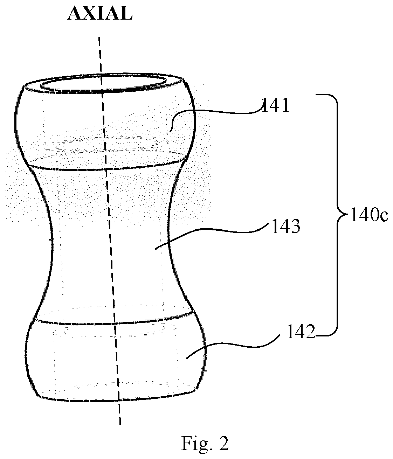

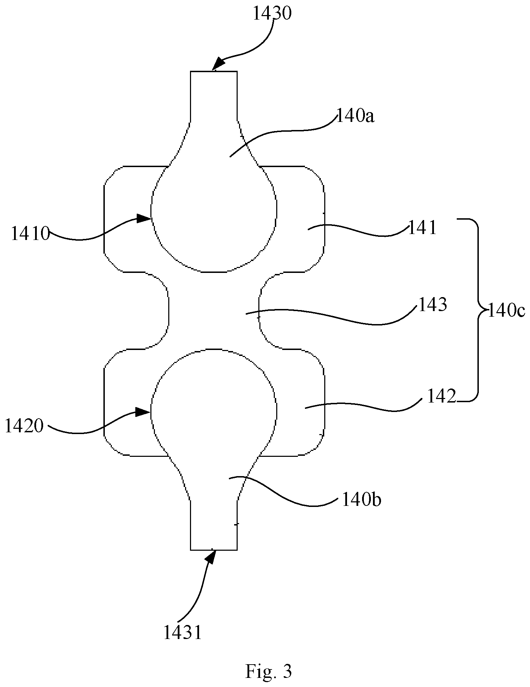

Reference is next made to FIG. 2 and FIG. 3, in this embodiment, the connecting member 140 includes a first element 140a, a second element 140b and a third element 140c, the first element 140a connected to the first frame 110 (more specifically, to the first fixation structure 111 of the first frame 110), the second element 140b connected to the second frame 120 (more specifically, to the second fixation structure 121 of the second frame 120), the first element 140a is rotatably connected to the third element 140c, the second element 140b is rotatably connected to the third element 140c. The third element 140c includes the first portion 141, second portion 142 and third portion 143 which connects the first portion 141 and second portion 142. Preferably, the third portion 143 has a radial dimension less than the first portion 141, and the third portion 143 has a radial dimension less than the second portion 142. For example, each of the first portion 141, second portion 142 and third portion 143 is a circular tube. In this case, the third portion 143 has a diameter less than the first portion 141 and the second portion 142. For another example, each of the first portion 141, second portion 142 and third portion 143 is a square tube. In this case, the third portion 143 has a side length less than the first portion 141 and second portion 142. In such a way, axial bending of the connecting member 140 is able to be facilitated.

In other embodiments, one or more of the first element 140a, the second element 140b and the third element 140c has at least a section comprising an arc section, a slot or a hollow structure to allow an axial bending. Preferably, the third portion 143 of the third element 140c may be provided with slots or hollow structures. For example, the third portion 143 of the third element 140c may be a slotted solid structure, which for example, has slots alternatively arranged at different angles to offer the third portion 143 of the third element 140c with a better axial bending.

Preferably, the connecting member 140 is made of soft materials. Preferably, one or more of the first element 140a, the second element 140b and the third element 140c is made of a biocompatible polymer material. For example, material of one or more of the first element 140a, the second element 140b and the third element 140c is at least one selected from the group consisting of polysulfone, polyphenylene sulfone, polyetheretherketone, elastic polyurethane, silicone rubber and polyethylene glycol.

In the first portion 141, there is a first receiving volume 1410. The first element 140a is received and movable in the first receiving volume 1410. Here, the first element 140a is rotatable in the first receiving volume 1410, allowing relative positional changes between the first frame 110 and the second frame 120. Similarly, in the second portion 142, there is a second receiving volume 1420. The second element 140b is received and movable in the second receiving volume 1420. Here, the second element 140b is rotatable in the second receiving volume 1420, allowing relative positional changes between the first frame 110 and the second frame 120. Further, the third portion 143 may be either solid or hollow.

Specifically, each of the first element 140a and the second element 140b is bulb-shaped. That is, each of the first element 140a and the second element 140b includes a spherical portion and a neck portion connected to the spherical portion. In this embodiment, the spherical portion of the first element 140a is received in the first receiving volume 1410, while the spherical portion of the second element 140b is received in the second receiving volume 1420. Additionally, one end of the neck portion of the first element 140a is connected to the spherical portion thereof while the other end of the neck portion of the first element 140a is fixedly connected to the first fixation structure 111. One end of the neck portion of the second element 140b is connected to the spherical portion thereof while the other end of the neck portion of the second element 140b is fixedly connected to the second fixation structure 121.

Further, the spherical portion of the first element 140a is rotatable within the first receiving volume 1410, and the spherical portion of the second element 140b is rotatable within the second receiving volume 1420. Specifically, the spherical portion of the first element 140a is rotatable within the first receiving volume 1410 about any axis in the X-Y plane and/or the Z-axis. Likewise, the spherical portion of the second element 140b is rotatable within the second receiving volume 1420 about any axis in the X-Y plane and/or the Z-axis. With continued reference to FIG. 3, as a result of a rotation of the spherical portion of the first element 140a within the first receiving volume 1410, the other end 1430 of the neck portion of the first element 140a will point to a different direction, which leads to a different configuration of the first frame 110 as a whole. Likewise, since the spherical portion of the second element 140b is rotatable within the second receiving volume 1420, the other end 1431 of the neck portion of the second element 140b orients to a different direction, so that the whole second frame 120 presents a different configuration.

In summary, when facing the left atrial appendages with different lumen shapes, the connecting member 140 allows changing the relative positional relationship between the first frame 110 and second frame 120, thereby enabling to modify the configuration of the left atrial appendage occluder 100 to accommodate various lumen shapes of left atrial appendage. Moreover, the relative positional relationship between the first frame 110 and second frame 120 is also able to be changed through the movable connection between the first fixation structure 111 and the connecting member 140 and the movable connection between the second fixation structure 121 and the connecting member 140, thereby enabling an additional adjustment of the configuration of the left atrial appendage occluder 100 to accommodate various lumen shapes of left atrial appendage.

Accordingly, in this embodiment, there is also provided a left atrial appendage occlusion system including a delivery catheter, a pusher and the left atrial appendage occluder 100 as defined above, the delivery catheter configured to establish a delivery channel, the pusher configured to push the left atrial appendage occluder 100 along the delivery channel. In practical use, the left atrial appendage occluder 100 is delivered by the pusher to a distal end of the delivery catheter and then released therefrom. During the release, the second frame 120 and the anchoring structure 130 thereon are pushed out from the delivery catheter and then positioned and secured to the inner wall of the left atrial appendage. After that, the first frame 110 is pushed out from the delivery catheter, and covers the opening of the left atrial appendage and well fits to the lumen shape of the left atrial appendage for achieving an optimal effect.

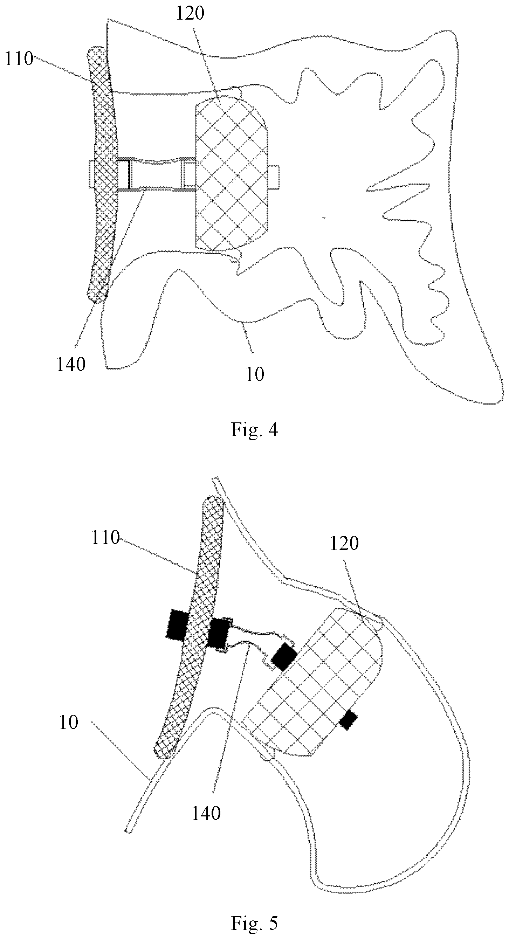

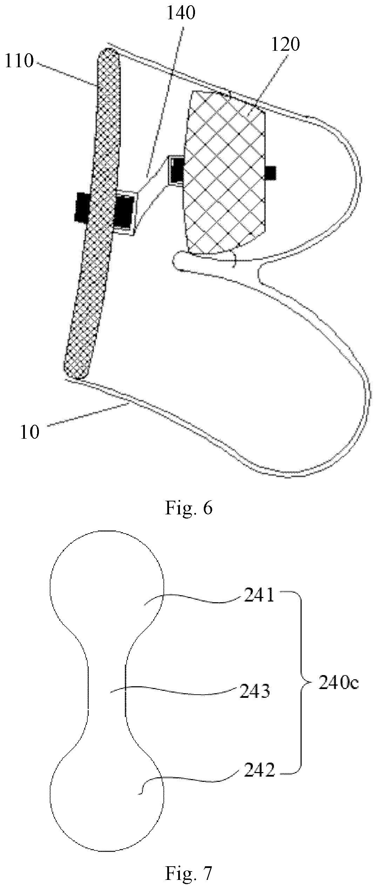

Accordingly, referring to FIG. 4 to FIG. 6, the left atrial appendage occluder 100 according to this embodiment can fit and adapt to various lumen shapes of the left atrial appendage very well, including normal shape, short chicken wing shape, multi-cavity shape, etc. As shown in FIG. 4 (optionally in combination with FIG. 1 to FIG 3), in case of a normal left atrial appendage, the left atrial appendage occluder 100 is able to well fit to the left atrial appendage 10 with substantially no deformation (i.e. no bending, in a natural configuration) of the connecting member 140. As shown in FIG. 5 (optionally in combination with FIG. 1 to FIG. 3), for a short chicken wing left atrial appendage, the left atrial appendage occluder 100 is able to well fit to the left atrial appendage 10 with a certain degree of axial bending of the connecting member 140. As shown in FIG. 6 (optionally in combination with FIG. 1 to FIG. 3), for a multi-cavity left atrial appendage, the left atrial appendage occluder 100 is able to well fit to the left atrial appendage 10 with a large axial bending of the connecting member 140. In this case, the well fit to the left atrial appendage 10 is achieved mainly through rotations of the first elements 140a and the second element 140b in the first receiving volume 1410 and the second receiving volume 1420, respectively To sum up, in the left atrial appendage occluder and left atrial appendage occlusion system according to this embodiment, the connecting member includes a first element, a second element and a third element, the first element connected to the first frame, the second element connected to the second frame, the first element rotatably connected to the third element, the second element rotatably connected to the third element. In such a design, when facing left atrial appendages with various shapes, relative position between the first frame and the second frame is able to be changed by the connecting member. That is, the configuration of the left atrial appendage occluder is able to be adjusted, so as to adapt various lumen shapes of the left atrial appendages.

### EMBODIMENT 2

Similarly, in this embodiment, the left atrial appendage occluder also includes: a first frame configured to delimit a substantially closed space with the left atrial appendage; a second frame, the exterior surface of which is attached with an anchoring structure; and a connecting member including a first element, a second element and a third element, the first element connected to the first frame, the second element connected to the second frame, the first element is rotatably connected to the third element, the second element is rotatably connected to the third element.

In embodiment 2, the first frame and the second frame can refer to the first frame 110 and the second frame 120 of embodiment 1. Further, connection between the first frame and the connecting member and connection between the second frame and the connecting member in embodiment 2 can also refer to connection between the first frame 110 and the connecting member 140 and connection between the second frame 120 and the connecting member 140 in embodiment 1, which may be a fixed connection or a movable connection. A repeated description thereof is omitted here.

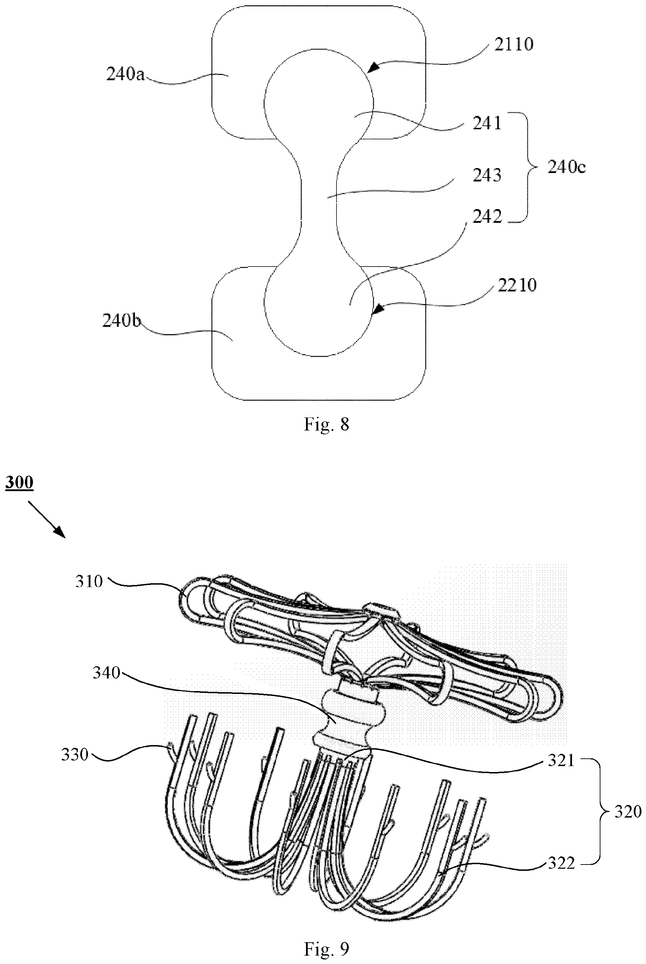

Embodiment 2 differs from embodiment 1 essentially in that: the connecting member according to embodiment 2 has a different structure from the connecting member according to embodiment 1. Specifically, referring to FIG. 7 and FIG. 8, the connecting member 240 according to embodiment 2 includes a first element 240a, a second element 240b and a third element 240c, the first element 240a connected to the first frame (not shown), the second element 240b connected to the second frame (not shown). The connection between the first element 240a and the third element 240c is rotatable. The connection between the second element 240b and the third element 240c is rotatable. In addition, the first element 240a forms a first receiving volume 2410, and the second element 240b forms a second receiving volume 2420. The opposing ends of the third element 240c are received in the first receiving volume 2410 and the second receiving volumes 2420, respectively. Here, the opposing ends of the third element 240c are respectively rotatable in the first receiving volume 2410 and the second receiving volumes 2420, thereby enabling to change relative position between the first frame and the second frame.

In this embodiment, the connecting member 240 is made of a soft material. Preferably, one or more of the first element 240a, the second element 240b and the third element 240c is made of a biocompatible polymer material. For example, the material of one or more of the first element 240a, the second element 240b and the third element 240c is at least one selected from the group consisting of polysulfone, polyphenylene sulfone, polyetheretherketone, elastic polyurethane, silicone rubber and polyethylene glycol.

Further, one or more of the first element 240a, the second element 240b and the third element 240c has at least a section comprising an arc section, a slot or a hollow structure to achieve an axial bending.

Preferably, each of a connecting section between the third element 240c and the first element 240a and a connecting section between the third element 240c and the second element 240b has greater radial dimension than a non-connecting section of the third element. Here, the third element 240c includes a first portion 241, a second portion 242 and a third portion 243 connecting the first portion 241 with the second portion 242. The first portion 241 is received in the first receiving volume 2410, and the second portion 242 is received in the second receiving volume 2420. The third portion 243 has a radial dimension less than the first portion 241, and the third portion 243 has a radial dimension less than the second portion 242.

Reference can be made to embodiment 1 for details in features that are not mentioned in embodiment 2, and a repeated description thereof is omitted here.

### EMBODIMENT 3

Referring to FIG. 9, a structural schematic diagram of a left atrial appendage occluder according to embodiment 3 of the present disclosure. Similarly, in this embodiment, the left atrial appendage occluder 300 includes: a first frame 310 configured to delimit a substantially closed space with the left atrial appendage; a second frame 320 including an anchoring structure 330; and a connecting member 340 including a first element, a second element and a third element (not shown), the first element connected to the first frame 310, the second element connected to the second frame 320, the first element is rotatably connected to the third element, the second element is rotatably connected to the third element.

With reference to FIG. 9, embodiment 3 differs from embodiments 1 and 2 in that: the second frame 320 in embodiment 3 has only one fixation structure. Specifically, the second frame 320 only includes a second fixation structure 321 and a plurality of second frame strips 322 fixedly attached to the second fixation structure 321. The second fixation structure 321 is located at a proximal end of the second frame 320, and each of the second frame strips 322 is hook-shaped. One end of each of second frame strips 322 is fixedly attached to the second fixation structure 321. In embodiment 3, the second frame 320 as a whole is umbrella-shaped.

In embodiment 3, the connecting member 340 can refer to the connecting member 140 in embodiment 1 and the connecting member 240 in embodiment 2. Accordingly, the first frame 310 can refer to the first frame 110 in embodiment 1. Connection between the first frame 310 and the connecting member 340 and connection between the second frame 320 and the connecting member 340 in embodiment 3 can also refer to connection between the first frame 110 and the connecting member 140 and connection between the second frame 120 and the connecting member 140 in embodiment 1, which may be a fixed connection or a movable connection. References can be made to embodiments 1 or 2 for details in features that are not mentioned in embodiment 3, and a repeated description thereof is omitted here.

### EMBODIEMNT 4

In this embodiment, the left atrial appendage occluder includes a first frame 410 configured to delimit a substantially closed space with the left atrial appendage to restrict the flow of blood into and out of the left atrial appendage; a second frame 420, the exterior surface of which is attached with an anchoring structure; and a connecting member 440 including a first element 440a and a second element 440b, the first element 440a connected to the first frame 410, the second element 440b connected to the second frame 420, the first element 440a is rotatably connected to the second element 440b.

The rotatable connection between the first element 440a and the second element 440b may be accomplished by a hinge or a combination of a ball head and an arcuate cavity as shown in FIG. 3. Each of the first element 440a and second element 440b may be implemented as a rigid or flexible rod. In a preferred embodiment, each of first element 440a and second element 440b is implemented as a connecting rod made of biocompatible polymer materials. The connecting rod has a central portion significantly thinner than its two end portions, i.e., forming a waisted shape. This helps a certain bending of the first element 440a or the second element 440b about respective central axes, so as to better adapt to various lumen shapes of left atrial appendages.

In embodiment 4, the first frame and the second frame can refer to the first frame 110 and the second frame 120 in embodiment 1. Further, connection between the first frame 410 and the connecting member 440 and connection between the second frame 420 and the connecting member 440 in embodiment 4 can also refer to connection between the first frame 110 and the connecting member 140 and connection between the second frame 120 and the connecting member 140 in embodiment 1, which may be a fixed connection or a movable connection, and the detailed description is omitted here. References can be made to embodiments 1, 2 or 3 for details in features that are not mentioned in embodiment 4, and a repeated description thereof is omitted here.

The description presented above is merely a few preferred embodiments of the present application and does not intend to limit the protection scope in any sense. Any changes and modifications made by those of ordinary skilled in the art based on the above teachings fall within the protection scope of the appended claims.

## Claims

1. A left atrial appendage occluder, comprising:
a first frame configured to delimit a substantially closed space with the left atrial appendage;
a second frame comprising an anchoring structure; and
a connecting member comprising a first element and a second element, the first element connected to the first frame, the second element connected to the second frame, the first element rotatably connected to the second element.

2. The left atrial appendage occluder of claim 1, wherein the first element forms a receiving volume, in which the second element is partially received to be rotatable relative to the first element; or the second element forms a receiving volume, in which the first element is partially received to be rotatable relative to the second element.

3. The left atrial appendage occluder of claim 2, wherein the receiving volume is a ball cup and the corresponding first or second element comprises a ball pin mating with the ball cup.

4. The left atrial appendage occluder of claim 1, wherein each or either of the first element and the second element is made of a biocompatible polymer material.

5. The left atrial appendage occluder of claim 1, wherein each or either of the first element and the second element has at least a section comprising an arc section, a slot or a hollow structure to allow an axial bending.

6. A left atrial appendage occluder, comprising:
a first frame configured to delimit a substantially closed space with the left atrial appendage;
a second frame having an anchoring structure fixed on an exterior surface thereof; and
a connecting member comprising a first element, a second element and a third element, the first element connected to the first frame, the second element connected to the second frame, the first element rotatably connected to the third element, the second element rotatably connected to the third element.

7. The left atrial appendage occluder of claim 6, wherein the third element forms a first receiving volume and a second receiving volume, the first element partially received in the first receiving volume, the second element partially received in the second receiving volume, each of the first element and the second element being rotatable with respect to the third element; or the first element forms a first receiving volume and the second element forms a second receiving volume, opposing ends of the third element received in the first receiving volume and the second receiving volume, respectively.

8. The left atrial appendage occluder of claim 6, wherein one or more of the first element, the second element and the third element is made of a biocompatible polymer material.

9. The left atrial appendage occluder of claim 4 or 8, wherein the biocompatible polymer material is at least one selected from the group consisting of polysulfone, polyphenylene sulfone, polyetheretherketone, elastic polyurethane, silicone rubber and polyethylene glycol.

10. The left atrial appendage occluder of claim 6, wherein one or more of the first element, the second element and the third element has at least a section comprising an arc section, a slot or a hollow structure to allow an axial bending.

11. The left atrial appendage occluder of claim 6, wherein each of a connecting section between the third element and the first element and a connecting section between the third element and the second element has a radial dimension greater than a non-connecting section of the third element.

12. The left atrial appendage occluder of claim 1 or 6, wherein a surface of the first frame is covered with a polymer membrane and/or a surface of the second frame is covered with a polymer membrane.

13. A left atrial appendage occlusion system, comprising: a delivery catheter; a pusher; and a left atrial appendage occluder as defined in any one of claims 1 to 12, the delivery catheter configured to establish a delivery channel, the pusher configured to push the left atrial appendage occluder along the delivery channel.
